# FASCICULE DE BREVET EUROPEEN

(11) **EP 1 433 496 B1**
(45) Date de publication et mention de la délivrance du brevet: **09.03.2016**
(21) Numéro de dépôt: 03293302.0
(22) Date de dépôt: 23.12.2003
(51) Int. Cl.: A61N 1/36, A61N 1/37

(54) **Dispositif médical actif, notamment dispositif implantable tel que stimulateur cardiaque, défibrillateur, cardioverteur ou dispositif multisite, comprenant des moyens de détection des troubles du sommeil**
Aktive medizinische Vorrichtung, insbesondere implantierbare Vorrichtung des Typs Herzschrittmachers, Defibrillators, Kardiovertierers oder Mehrstelleneinrichtung, mit Mitteln zur Erkennung von Schlafstörungen
Active medical device, in particular implantable device such as pacemaker, defibrillator, cardioverter or multisite device, having means for detecting sleep disorders

(30) Priorité: 24.12.2002 FR 0216608
(43) Date de publication de la demande: 30.06.2004
(73) Titulaire: ELA MEDICAL, 92120 Montrouge (FR)
(72) Inventeur: Poezevara, Yann, 91080 Courcouronnes (FR)
(74) Mandataire: Dupuis-Latour, Dominique

(56) Documents cités:
- EP-A- 0 940 155
- EP-A- 1 151 718
- EP-A- 1 151 719

## Description

L'invention concerne le diagnostic des troubles respiratoires du sommeil, et plus particulièrement le diagnostic des épisodes d'apnée ou d'hypopnée.

L'invention vise, entre autres, à diagnostiquer des affections telles que le syndrome d'apnée du sommeil (SAS), apnée obstructive ou bien centrale, qui est une pathologie susceptible d'entraîner un certain nombre de troubles tels qu'hypersomnolence diurne, trouble du rythme cardiaque, hypertension.

Un SAS peut être défini par une récurrence importante d'apnées ou d'hypopnées associées à des signes cliniques. Une "apnée" ou "pause respiratoire" est un arrêt temporaire de la fonction respiratoire de durée supérieure à 10 secondes et survenant pendant une phase de sommeil du patient ; une "hypopnée" est définie comme une décroissance importante de la ventilation-minute, par exemple plus de 50%, par rapport à une moyenne de référence antérieure, mais sans arrêt de la fonction respiratoire (la ventilation-minute est le produit de l'amplitude par la fréquence des cycles respiratoires successifs).

On connaît diverses techniques pour détecter ces troubles respiratoires du sommeil, techniques qui mettent en oeuvre divers types de capteurs, notamment un capteur de ventilation-minute, paramètre à prépondérance physiologique obtenue par une mesure intrathoracique d'impédance opérée avec un dispositif implanté.

La mesure de la ventilation-minute est réalisée par injection d'impulsions d'un courant constant de quelques centaines de microampères à une fréquence de quelques hertz entre deux électrodes disposés dans la cage thoracique ; l'injection peut également avoir lieu entre le boîtier du dispositif médical implanté et une électrode, par exemple une électrode de stimulation si le dispositif implanté est un dispositif cardiaque. Les variations d'impédance reproduisent les variations du volume thoracique, avec des pics d'impédance lors de l'inspiration, lorsque les poumons sont remplis d'air, et une impédance décroissante lors de la phase expiratoire.

D'autre techniques ont été proposées, toujours basées sur la mesure d'un paramètre à prépondérance physiologique tel que la saturation en oxygène dans le sang : la réduction prolongée de l'activité respiratoire produit une désaturation corrélative en oxygène, qui peut être détectée par un capteur approprié, par exemple placé à l'extrémité d'un cathéter ou d'une sonde d'un stimulateur cardiaque.

Il a été toutefois constaté en pratique, lors d'études cliniques, qu'un système mettant en oeuvre un capteur d'activité respiratoire enregistrant les variations du volume pulmonaire au niveau thoracique peut être dans certaines circonstances leurré au cours du sommeil pour des raisons :
- physiologiques internes, par exemple une position corporelle ou un mouvement induisant une modification des volumes et/ou de la position des organes concernés,
- cliniques, par exemple du fait d'une respiration d'origine exclusivement abdominale, ou
- externes, par exemple du fait de perturbations électromagnétiques passagères.

Dans ces situations particulières, ce dispositif détecte - à tort - la survenue d'une apnée ou d'une hypopnée, alors qu'en réalité la respiration est normale mais n'as été diagnostiquée comme telle par le dispositif.

De même, une apnée ou une hypopnée peut provoquer un micro-réveil sans pour autant affecter de façon significative la saturation en oxygène dans le sang.

Le EP 1 151 719 A2 décrit d'autres techniques de caractérisation d'un trouble respiratoire, dont certaines consistent à analyser le rythme cardiaque, en surveillant la modulation de la moyenne de la fréquence cardiaque, cette modulation résultant en fait de l'effet mécanique de la respiration sur le thorax.

D'autres techniques encore sont exposées dans les EP 0 940 155 A2 (notamment la détection d'une bradycardie, révélée par le franchissement d'un seuil) et EP 1 151 718 A2 (analyse spectrale du rythme cardiaque, notamment).

L'invention a pour but d'éliminer ces risques de faux positifs et de faux négatifs dans les dispositifs médicaux pourvu des moyens de détection des troubles respiratoires, notamment les dispositifs médicaux implantés.

Le point de départ de départ de l'invention réside dans l'observation clinique du fait que, en réponse à l'hypoxie engendrée par les apnées ou les hypopnées et en l'absence de flux respiratoire (poumons non étirés), les chémorécepteurs carotidiens promeuvent un effet vagotonique sur le coeur de sorte que, en fonction de l'état de la balance sympathico-vagale, les apnées ou hypopnées peuvent induire une bradycardie. Puis, à la fin de l'épisode apnéique ou hypopnéique, la reprise ventilatoire - qui peut être consécutive à un micro-réveil ou à une modification de la régulation de la pression partielle en CO₂ dans le sang - s'accompagne presque systématiquement d'une tachycardie sous l'effet de l'augmentation du tonus sympathique sur le coeur. Cette augmentation résulte elle-même de plusieurs facteurs tels que conséquences d'un micro-réveil, détection de l'hypoxie par les chémorécepteurs périphériques, ou coactivation des neurones du système sympathique cardiovasculaire par les centres de la respiration.

Ainsi, la répétition des apnées ou hypopnées confère à la fréquence cardiaque un aspect cyclique avec alternance d'épisodes de bradycardie/tachycardie qui peuvent être caractéristiques.

Le dispositif proposé par l'invention est un dispositif médical actif, notamment un "dispositif médical implantable actif' selon la directive 90/385/ CEE du 20 juin 1990 du Conseil des communautés européennes, et plus particulièrement un dispositif tel que stimulateur cardiaque, défibrillateur, cardioverteur et/ou dispositif multisite du type décrit par le EP 1 151 719 A2 précité, correspondant au préambule de la revendication 1. Les éléments caractéristiques de l'invention sont énoncés dans la partie caractérisante de cette revendication.

Des formes de réalisation particulières sont énoncées dans les sous-revendications.

On va maintenant décrire de façon plus détaillée diverses manières de mettre en oeuvre l'invention.

L'invention peut notamment être appliquée à des appareils tels que les dispositifs implantés commercialisés par ELA Médical, Montrouge, France. Il s'agit de dispositifs à microprocesseur programmables comportant des circuits pour recevoir, mettre en forme et traiter des signaux électriques recueillis par des électrodes implantées. Il est possible d'y transmettre par télémétrie des logiciels qui seront implantés en mémoire et exécutés pour mettre en oeuvre les fonctions de l'invention décrites ci-dessous. L'adaptation de ces appareils à la mise en oeuvre des fonctions de l'invention est à la portée de l'homme du métier, et elle ne sera pas décrite en détail.

Comme on l'a indiqué plus haut, la présente invention propose d'utiliser un dispositif médical actif implantable pour caractériser des épisodes de troubles respiratoires du sommeil par analyse des variations de la fréquence cardiaque ou de la variabilité sinusale, de manière à pouvoir réaliser de façon automatique et continue une détection et un diagnostic des troubles respiratoires présentés par le patient. Ce diagnostic peut être opéré directement à partir de l'analyse du rythme cardiaque.

En variante ou en complément, pour améliorer la spécificité globale de ce diagnostic, il est possible d'utiliser l'analyse du rythme cardiaque effectuée pour la caractérisation des troubles respiratoires du sommeil, comme information complémentaire à celle délivrée par un autre capteur, par exemple un capteur de ventilation-minute (capteur MV). Dans ce dernier cas, le dispositif de l'invention peut par exemple, à partir d'une suspicion d'apnée détectée par le capteur MV, valider cet épisode ou, inversement, suspecter une apnée qui sera validée par le capteur MV.

Outre la détection proprement dite des troubles respiratoires du sommeil (apnées et hypopnées), l'étude des variations du rythme cardiaque permet de caractériser la réactivité du système sympathique du patient. Cette information peut notamment, sur le long terme, servir à observer l'effet d'un traitement médicamenteux tel que l'administration d'un bêtabloqueur, ou l'évolution d'une pathologie telle qu'une insuffisance cardiaque.

De façon générale, pour atteindre les buts précités, l'appareil surveille en permanence les signaux cardiaques de manière à déterminer la fréquence sinusale. Pour cela, l'appareil peut déterminer l'intervalle PP (entre deux ondes auriculaires spontanées) ou l'intervalle RR (entre deux complexes ventriculaires QRS spontanés), la variabilité cycle à cycle de l'intervalle PR étant considérée comme négligeable.

Le dispositif ne retient que les signaux correspondant à une activité spontanée régulière. Pour l'analyse du rythme, le dispositif détecte et élimine les extrasystoles et les salves auriculaires ou ventriculaires, qui modifient instantanément la fréquence cardiaque sans nécessairement être la conséquence de variations trouvant leur origine dans le système nerveux autonome. Le dispositif élimine également les cycles cardiaques stimulés, car dans ce cas le rythme est un rythme stable mais provoqué.

Après avoir sélectionné de la sorte les seuls intervalles PP ou RR significatifs, le dispositif analyse les cycles ainsi mémorisés pour détecter des éventuels épisodes de trouble respiratoire du sommeil.

Pour cela, deux techniques peuvent être mise en oeuvre :
- l'analyse du profil de variation de la fréquence cardiaque, afin d'identifier des profils cycliques d'épisodes bradycardie/tachycardie, ou
- l'analyse de la variabilité sinusale, ce paramètre reflétant les variations de la fréquence cardiaque sur la période donnée.

### Caractérisation d'un épisode d'apnée ou d'hypopnée par analyse du profil de la fréquence cardiaque

Si l'appareil est doté d'un ou plusieurs capteurs permettant de suspecter un trouble respiratoire du sommeil, par exemple un capteur de ventilation-minute opérant par mesure de l'impédance transthoracique, d'une impédance intracardiaque (cf. les EP-A-1 116 497 et EP-A-1 138 346 au nom de ELA Medical) ou de la PEA (*Peak Endocardial Acceleration*), le dispositif peut évaluer le retentissement de ce trouble respiratoire sur la fréquence cardiaque, et ce à plusieurs fins, notamment :
- la validation de la détection d'un épisode respiratoire pathologique (apnée ou hypopnée par exemple),
- la caractérisation de l'impact de l'événement sur la fréquence cardiadiaque (existence ou non d'une alternance bradycardie/tachycardie, amplitude et durée de la bradycardie et de la tachycardie), etc.,
- le déclenchement instantané d'une réaction de l'appareil : mémorisation d'autres informations (par exemple d'un ECG intracardiaque) ou mise en oeuvre d'une thérapie visant à prévenir d'éventuelles nouvelles phases de bradycardie/tachycardie, notamment en maintenant une fréquence de stimulation plus élevée.

Dans le cas particulier du PEA, on sait recueillir un signal d'accélération endocardiaque, comme cela est par exemple décrit dans les EP-A-0 515 319 et EP-A-0 655 260 (Sorin Biomedica Cardio SpA) qui décrit une sonde endocavitaire pourvue d'une électrode distale de stimulation implantée au fond du ventricule, intégrant en outre un micro-accéléromètre permettant de mesurer l'accélération endocardiaque. Un dispositif correspondant est commercialisé par la société Sorin Biomedica Cardio SpA sous la dénomination *Living CHF,* avec une électrode de type *Best.* Le signal d'accélération endocardiaque ainsi mesuré au cours d'un cycle cardiaque forme, entre autres, deux pics correspondant aux deux bruits majeurs qu'il est possible de reconnaître dans chaque cycle d'un coeur sain. Le EP-A-0 655 260 précité décrit une manière de traiter le signal d'accélération endocavitaire délivré par le capteur situé en bout de sonde pour en dériver, notamment, ces deux valeurs de pic d'accélération endocardiaque, utiles notamment pour la détection de troubles cardiaques et le déclenchement ou non d'une thérapie de défibrillation.

Dans l'exemple où l'appareil est doté d'un capteur d'impédance transthoracique capable d'identifier une apnée de sommeil, lorsque ce capteur détecte une apnée, le dispositif analyse de la manière suivante le profil de la fréquence cardiaque.
a) prise en compte des n cycles cardiaques précédant la détection de l'apnée et des m cycles suivant cette détection ;
b) calcul de la variation cycle à cycle de l'intervalle cardiaque (PP ou RR) ;
c) lorsque la variation atteint un seuil positif prédéterminé, détection d'un début de bradycardie ;
d) lorsque la variation change de signe, détection d'un début de tachycardie ;
e) mémorisation de la durée de la bradycardie et de la valeur minimum de la fréquence cardiaque ;
f) lorsque la variation se stabilise, détection de la fin de la tachycardie ;
g) mémorisation de la durée de la tachycardie et de la valeur maximum de la fréquence cardiaque ;
h) si les caractéristiques détectées et mémorisées de la bradycardie et de la tachycardie remplissent des critères prédéterminés (durée, seuil de fréquence, etc.), validation de la détection de l'épisode d'apnée.

On notera que, pour permettre une analyse en temps réel, certaines étapes du calcul peuvent être anticipées, par exemple le précalcul de la variation cycle à cycle de l'intervalle et la détermination du sens et de l'importance de cette variation.

### Caractérisation du profil respiratoire du patient par analyse de la variabilité sinusale

Dans ce cas, c'est la variabilité sinusale qui est analysée, seule ou en complément de tout autre paramètre, pour déterminer la présence ou l'absence d'un trouble respiratoire du sommeil.

À partir des données de rythme cardiaque enregistrées, un indice de variabilité sinusale est calculé, dans le domaine temporel ou dans le domaine fréquentiel. Ce calcul peut être opéré soit en interne au sein de l'appareil implanté, soit par un dispositif externe qui reçoit les informations recueillies et enregistrés par l'appareil implanté.

Le calcul peut être effectué pour une tranche horaire déterminée et/ou en fonction d'événements détectés par l'appareil implanté. Par exemple, si l'appareil est équipé d'un moyen de détection du sommeil du patient, l'indice de variabilité sinusale peut être calculé séparément pour les phases de veille et les phases de sommeil. On pourra se référer à cet égard au EP-A-0 970 713 (ELA Médical), qui décrit un dispositif implanté pourvu de moyens de discrimination des phases d'éveil et de sommeil.

En effet, dans le cas d'un patient sain, l'indice varie peu entre le jour et la nuit, à la différence d'un patient apnéique où cet indice présente des variations significativement plus importantes.

Si l'indice de variabilité sinusale de nuit atteint un seuil prédéterminé, ou bien si le rapport des indices nuit/jour atteint un seuil prédéterminé, on peut conclure que la variabilité sinusale est significativement élevée durant les phases de sommeil. La valeur seuil permettra de discriminer les patients sains des patients atteints de troubles respiratoires du sommeil, ou risquant de présenter de tels troubles.

À plus long terme, l'évolution dans le temps de l'indice de variabilité sinusale peut être mémorisé, par exemple calculé et mémorisé toutes les 24 heures, seul ou en complément de l'information délivrée par d'autres capteurs, ceci afin d'évaluer l'efficacité d'un traitement (par exemple traitement d'une apnée obstructive par instauration d'une pression positive continue) ou l'évolution d'un trouble respiratoire du sommeil (par exemple lié à un prise de poids importante).

Des nombreux indices, en eux-mêmes connus, peuvent être choisis pour représenter la variation sinusale.

On peut utiliser les indices suivants, calculés par analyse dans le domaine temporel :
- PNN₅₀ (*Percentage Normal-to-Normal*) qui est le pourcentage de cycles dont la variation cycle à cycle est supérieure à 50 ms,
- RMSSD, qui est la racine carrée du rapport entre, d'une part, la somme des carrés des différences de durée de deux cycles consécutifs et, d'autre part, le nombre total de cycles analysés, ou
- SDNN (*Standard Deviation Normal-to-Normal*), qui est l'écart-type des intervalles des cycles cardiaques.

Dans le domaine fréquentiel il est par exemple possible, après analyse spectrale des intervalles des cycles cardiaques par une transformation rapide de Fourier et détermination de bandes spectrales, de calculer le rapport des puissances entre les fréquences basses (par exemple sur la plage 0,02 Hz - 0,15 Hz) et les fréquences élevées (par exemple supérieures à 0,2 Hz).

Pour une application en temps réel, on choisit de préférence un indice de variabilité sinusale du domaine temporel.

Si l'on choisit par exemple l'indice SDNN, la caractérisation d'un épisode d'apnée peut alors être opérée de la manière suivante :
- périodiquement, calcul de l'indice de variabilité sinusale en l'absence de suspicion d'apnée ou hypopnée, de manière à constituer un indice de référence ; si l'appareil est équipé d'un moyen de détection du sommeil du patient, l'indice de référence est avantageusement calculé séparément pour les phases d'éveil et pour les phases de sommeil. On peut ainsi par exemple calculer SDNN sur 30 cycles cardiaques toutes les 10 minutes (ou tous les 150 cycles cardiaques), et mémoriser les valeurs moyennes minimum et maximum du SDNN pour servir de référence.
- lorsqu'une apnée ou une hypopnée est suspectée par un autre capteur (par exemple un capteur de ventilation-minute), le SDNN courant est calculé sur les cycles précédant et suivant cette détection, par exemple les 20 cycles précédant et les 10 cycles suivant la détection de suspicion d'apnée ou d'hypopnée.
- si la SDNN courante est significativement plus élevée que la SDNN de référence, validation de la détection de l'épisode d'apnée ou d'hypopnée - par exemple, si la SDNN courante est supérieure à x fois la SDNN de référence, ou bien supérieure à x fois la valeur maximale de la SDNN de référence.

## Revendications

1. Un dispositif médical implantable actif du type stimulateur cardiaque, défibrillateur, cardioverteur et/ou dispositif multisite, comprenant :
- des moyens de recueil du rythme cardiaque dans au moins une cavité du coeur ;
- des moyens d'analyse du rythme, comprenant des moyens pour évaluer des variations de la fréquence cardiaque dans le temps en fonction du rythme ainsi recueilli ; et
- des moyens pour déterminer la présence d'un trouble respiratoire du sommeil en fonction de ces variations, ces moyens opérant cycle à cycle et étant aptes à :
· discriminer dans le rythme recueilli les événements spontanés et d'origine sinusale, en éliminant les extrasystoles, les salves auriculaires ou ventriculaires et les cycles cardiaques stimulés ; et
· déterminer, pour les événements spontanés d'origine sinusale ainsi discriminés, l'intervalle PP entre deux ondes auriculaires spontanées, ou l'intervalle RR entre deux complexes ventriculaires spontanés.
**caractérisé en ce que** les moyens d'analyse du rythme sont des moyens aptes à déterminer un indice de variabilité sinusale fonction desdites variations, et à comparer cet indice à un seuil prédéterminé.

2. Le dispositif de la revendication 1, dans lequel ledit indice de variabilité sinusale est un indice calculé dans le domaine temporel, choisi dans le group formé par : le pourcentage PNN₅₀ de cycles dont la variation cycle à cycle est supérieure à 50 ms ; la racine carrée RMSSD du rapport entre la somme des carrés des différences de durée de deux cycles consécutifs et le nombre total de cycles analysés ; et l'écart-type SDNN des intervalles des cycles cardiaques.

3. Le dispositif de la revendication 1, dans lequel ledit indice de variabilité sinusale est un indice calculé dans le domaine fréquentiel, fonction du rapport des puissances entre des fréquences basses et des fréquences élevées, déterminées après analyse spectrale des intervalles des cycles cardiaques.

4. Le dispositif de la revendication 1, comprenant en outre des moyens de mémorisation dudit indice de variabilité sinusale.

5. Le dispositif de la revendication 1, comprenant en outre au moins un capteur physiologique apte à délivrer un signal de mesure de l'activité respiratoire du patient, et des moyens pour corréler les indications de ce capteur physiologique avec celles desdits moyens d'analyse du rythme.

6. Le dispositif de la revendication 5, dans lequel lesdits moyens pour corréler les indications du capteur physiologique avec celles des moyens d'analyse du rythme comprennent des moyens aptes à confirmer un état d'apnée en fonction du résultat de la corrélation.

## Patentansprüche

1. Aktive implantierbare medizinische Vorrichtung vom Typ eines Herzstimulators, Defibrillators, Kardioverters und/oder einer Mehrstellenvorrichtung, welche aufweist:
- Mittel zur Erfassung des Herzrhythmus in wenigstens einem Hohlraum des Herzens;
- Mittel zur Analyse des Rhythmus, welche Mittel zur Auswertung der zeitlichen Schwankungen der Herzfrequenz in Abhängigkeit von dem so erfassten Rhythmus beinhalten; und
- Mittel zum Bestimmen des Vorliegens einer Schlafatemstörung in Abhängigkeit von diesen Schwankungen, wobei diese Mittel zyklusweise arbeiten und in der Lage sind:
· in dem erfassten Rhythmus die spontanen Ereignisse sinusalen Ursprungs zu diskriminieren, durch Eliminieren der Extrasystolen, der aurikulären oder ventrikulären Salven und der stimulierten Herzzyklen; und
· für die so diskriminierten spontanen Ereignisse sinusalen Ursprungs das PP-Intervall zwischen zwei spontanen aurikulären Wellen oder das RR-Intervall zwischen zwei spontanen ventrikulären Komplexen zu bestimmen,
**dadurch gekennzeichnet, dass** die Mittel zur Analyse des Rhythmus Mittel sind, die in der Lage sind, einen Index der Sinusvariabilität zu bestimmen, der von diesen Schwankungen abhängig ist, und diesen Index mit einem vorbestimmten Schwellenwert zu vergleichen.

2. Vorrichtung nach Anspruch 1, wobei der Index der Sinusvariabilität ein im Zeitbereich berechneter Index ist, der aus der Gruppe ausgewählt ist, welche aus Folgenden besteht: dem Prozentsatz PNN₅₀ von Zyklen, deren Variation von Zyklus zu Zyklus größer als 50 ms ist; der Quadratwurzel RMSSD des Verhältnisses zwischen der Summe der Quadrate der Differenzen der Dauer von zwei aufeinander folgenden Zyklen und der Gesamtzahl der analysierten Zyklen; und der Standardabweichung SDNN der Intervalle der Herzzyklen.

3. Vorrichtung nach Anspruch 1, wobei der Index der Sinusvariabilität ein im Frequenzbereich berechneter Index ist, der eine Funktion des Verhältnisses der Leistungen zwischen niedrigen Frequenzen und hohen Frequenzen ist, die nach einer Spektralanalyse der Intervalle der Herzzyklen bestimmt wurden.

4. Vorrichtung nach Anspruch 1, welche außerdem Mittel zur Speicherung des Index der Sinusvariabilität aufweist.

5. Vorrichtung nach Anspruch 1, welche außerdem wenigstens einen physiologischen Sensor, der in der Lage ist, ein Messsignal der Atmungsaktivität des Patienten zu liefern, und Mittel zum Korrelieren der Angaben dieses physiologischen Sensors mit denjenigen der Mittel zur Analyse des Rhythmus aufweist.

6. Vorrichtung nach Anspruch 5, wobei die Mittel zum Korrelieren der Angaben des physiologischen Sensors mit denjenigen der Mittel zur Analyse des Rhythmus Mittel aufweisen, die in der Lage sind, in Abhängigkeit von dem Ergebnis der Korrelation einen Apnoe-Zustand zu bestätigen.

## Claims

1. An active implantable medical device, such as a pacemaker, defibrillator, cardioverter and/or multisite device, comprising:
- means for collecting the cardiac rhythm in at least one cavity of the heart;
- means for analysing the rhythm, comprising means for evaluating variations of the cardiac frequency over time as a function of the so-collected rhythm; and
- means for determining the presence of a sleep breathing trouble as a function of these variations, these means operating on a cycle basis and being adapted to:
. discriminate in the collected rhythm the spontaneous events of sinusal origin, by eliminating the extrasystoles, the atrial or ventricular bursts and the paced cardiac cycles; and
. determining, for the so-discriminated spontaneous events of sinusal origin, the interval PP between two spontaneous atrial waves, or the interval RR between two spontaneous ventricular complexes,
**characterized in that** the rhythm analysis means are means adapted to determine an index of sinusal variability function of said variations, and to compare this index with a predetermined threshold.

2. The device of claim 1, wherein said index of sinusal variability is an index calculated in the time domain, chosen in the group formed by: the percentage PNN₅₀ of cycles whose cycle-to-cycle variation is higher than 50 ms; the square root RMSSD of the ratio between the sum of the squares of the differences of duration of two consecutive cycles and the total number of cycles analysed; and the standard deviation SDNN of the intervals of the cardiac cycles.

3. The device of claim 1, wherein said index of sinusal variability is an index calculated in the frequential domain, function of the ratio of the powers between low and high frequencies, determined after a spectral analysis of the cardiac cycle intervals.

4. The device of claim 1, further comprising memorising means of said index of sinusal variability.

5. The device of claim 1, further comprising at least one physiological sensor adapted to deliver a signal of measurement of the patient's respiratory activity, and means for correlating the indications of this physiological sensor with those of said rhythm analysis means.

6. The device of claim 5, wherein said means for correlating the indications of the physiological sensor with those of the rhythm analysis means comprise means adapted to confirm a state of apnoea as a function of the result of the correlation.
